(19) Europäisches Patentamt
European Patent Office
Office européen des brevets

(11) **EP 4 563 078 B1**

(12) **EUROPEAN PATENT SPECIFICATION**

(45) Date of publication and mention
of the grant of the patent:
**14.01.2026 Bulletin 2026/03**

(21) Application number: **24203073.2**

(22) Date of filing: **27.09.2024**

(51) International Patent Classification (IPC):
*A61B 5/00* (2006.01)   *G16H 50/20* (2018.01)
*G06N 3/045* (2023.01)   *G16H 20/10* (2018.01)
*G16H 20/30* (2018.01)   *G16H 40/63* (2018.01)
*G16H 40/67* (2018.01)   *G16H 50/70* (2018.01)
*G16H 80/00* (2018.01)   *G16H 50/30* (2018.01)
*G06N 3/04* (2023.01)

(52) Cooperative Patent Classification (CPC):
**A61B 5/4824; A61B 5/7267; G06N 3/045;
G16H 20/10; G16H 20/30; G16H 40/63;
G16H 40/67; G16H 50/20; G16H 50/30;
G16H 50/70; G16H 80/00;** G06N 3/04; G16H 50/50

(54) **GENERATING TINY DQN MODELS WITH OPTIMAL SET OF SENSORS FOR WEARABLE DEVICE-BASED PAIN ASSESSMENT**

ERZEUGUNG VON KLEINEN DQN-MODELLEN MIT OPTIMALEM SATZ VON SENSOREN ZUR SCHMERZBEURTEILUNG AUF DER BASIS EINER WEARABLE-VORRICHTUNG

GÉNÉRATION DE MODÈLES DQN MINUSCULES AVEC UN ENSEMBLE OPTIMAL DE CAPTEURS POUR UNE ÉVALUATION DE DOULEUR BASÉE SUR UN DISPOSITIF PORTABLE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **28.11.2023 IN 202321080711**

(43) Date of publication of application:
**04.06.2025 Bulletin 2025/23**

(73) Proprietor: **Tata Consultancy Services Limited
Maharashtra (IN)**

(72) Inventors:
• **MUKHOPADHYAY, SHALINI**
**700091 Kolkata, West Bengal (IN)**
• **SINHA, ANIRUDDHA**
**700091 Kolkata, West Bengal (IN)**
• **DEY, SWARNAVA**
**700091 Kolkata, West Bengal (IN)**
• **GHOSE, AVIK**
**700091 Kolkata, West Bengal (IN)**

(74) Representative: **Goddar, Heinz J.
Boehmert & Boehmert
Anwaltspartnerschaft mbB
Pettenkoferstrasse 22
80336 München (DE)**

(56) References cited:
**US-A1- 2022 160 296    US-A1- 2023 334 330**

• **PHAM NHAT ET AL: "PROS an efficient pattern-driven compressive sensing framework for low-power biopotential-based wearables with on-chip intelligence", PROCEEDINGS OF THE GENETIC AND EVOLUTIONARY COMPUTATION CONFERENCE, ACMPUB27, NEW YORK, NY, USA, 14 October 2022 (2022-10-14), pages 661 - 675, XP059276168, ISBN: 978-1-4503-9268-6, DOI: 10.1145/3495243.3560533**
• **LI YI ET AL: "LSTM-DNN based Approach for Pain Intensity and Protective Behaviour Prediction", 2020 15TH IEEE INTERNATIONAL CONFERENCE ON AUTOMATIC FACE AND GESTURE RECOGNITION (FG 2020), IEEE, 16 November 2020 (2020-11-16), pages 819 - 823, XP033879851, DOI: 10.1109/FG47880.2020.00061**

**Description**

CROSS-REFERENCE TO RELATED APPLICATIONS AND PRIORITY

The present application claims priority to Indian application no. Indian Patent Application No. 202321080711, filed on November 28, 2023

TECHNICAL FIELD

The embodiments herein generally relate to the field of machine learning based health state detection and, more particularly, to a method and system for generating tiny Deep Q-Network (DQN) models with optimal set of sensors for wearable device-based pain assessment.

BACKGROUND

Sensors are regularly used to understand various physiological markers for health monitoring purposes. In the past decade, wearable sensors have been used for unobtrusive sensing of various physiological as well as psychological phenomena of the human system. Wearable devices can be utilized for various health and wellness applications, among which detection and analysis of emotion, stress, and physical activity may constitute 'affective sensing'. An 'affective wearable' can be described as a wearable device equipped with sensors and systems enabling sensing of the user's affective behavior patterns, which also include facial expressions, gestures, voice features, as well as physiological markers such as heart rate, skin conductance, etc. The study on affective wearables can also be extended to pain detection and rehabilitation, to ensure proper dosage of medication, in turn leading to prevention of abuse of pain medication. This includes sensing and detection of presence and intensity of pain, as well as behavioral changes due to pain or fear of pain. Pain, like other affective outcomes, is an integrative phenomenon coupled with dynamic interactions between the brain's contextual, as well as sensory processes, which are often associated with detectable neuro-physiological changes. Recent advances in Artificial Intelligence (AI) and Machine Learning (ML) tools and technologies have enabled the exploration of neuro-computation techniques for different types of pain detection. This includes acute and chronic pain, and detection of pain levels or intensity of pain, and its improvement when under medication. Sensing techniques include electrocardiogram (ECG), electroencephalogram (EEG), galvanic skin resistance (GSR) and electromyogram EMG and the like. There are several causes of pain which require a long duration of medication and rehabilitation, be it due to environmental factors, injuries, or underlying diseases such as sickle cell disease. Accurate detection of existence of pain, as well as pain levels is crucial for any of the above-mentioned scenarios. This can be performed in a regular checkup-like frequency (via EEG wearable) or in unobtrusive continuous monitoring scenarios, in which case, ECG, GSR and EMG sensors can be used. There are several challenges involved in the domain, some of which are listed below:

- Signal corruptions in Free-living conditions
- Availability of sufficient data with pain annotation
- A robust model correlating with actual pain administered.
- Need for tiny models to run on the embedded device and provide low-latency real-time feedback.
- Selection and grading of sensor for pain quantification.
- Addressing the variability among individuals (baselining)

Pain and stress are interconnected in many ways and the intertwined relations between these two mechanisms has been established in previous works. Pain has multiple contributing factors which are behavioral, psychological, as well as social and stress is caused due to emotional strain and psychological stress along with other related reactions causes disruptions in the balance of various physiological processes. The cause of stress may be the physiological effect of the pain itself, or the consequences of pain in terms of psychological reasons. Acute and chronic Pain are quite stressful enough, especially if it is intense or long lasting. For instance, people suffering from back pain could easily develop stress by further induced muscle tension or spasms. On the other hand, the consequences of ongoing pain usually last longer than half a year and this kind of chronic pain would have a greater impact to the patient's quality of life. There are staggered research works in Affective wearables, but none in much depth, to study and establish the link between the affective computing and pain level detection. Existing works in literature use Heart rate variability HRV, some, electrodermal activity EDA, EEG, EMG, facial image/video, or voice analysis to monitor different forms and expressions of pain. This comes hand-in-hand with the research on affective computing, where emotion and stress estimation are performed. There are few works on pain detection from EEG. Other solutions include pain detection from visual data or Electro-dermal activity or Heart Rate variability. Data used was of thermally and electrically induced pain.

However state-of-the-art approaches focus on offline analysis and hardly address real-time detection of pain. Further, combination of sensors is hardly focused. While the effect of these affective patterns is seen in some physiological signals, the cause and origin can be detected from other sensor inputs. Thus, the features and indications extracted and the signature due to manifestation of each form of pain changes, resulting in change of the optimal model architecture.

Straight forward approach to combine multitude of sensor data for pain assessment does not help. The first thing is that this increases data to be analyzed by ML models adding to higher computational capacity on wearable devices. Furthermore, combining multiple sensors data is useful only if the right combination of sensors from available sensors are intelligently chosen in accordance with pain type. Simply including all sensors can add redundant inputs. is a critical decision and technically challenging as it is not a straight forward sensor to pain mapping. Document PROS: an Efficient Pattern-Driven Compressive Sensing Framework for Low-Power Biopotential-based Wearables with On-chip Intelligence" (D1) discloses PROS, an efficient pattern-driven compressive sensing framework for low-power biopotential-based wearables. PROS eliminates the conventional trade-off between signal quality, response time, and power consumption by introducing tiny pattern recognition primitives and a pattern-driven compressive sensing technique that exploits the sparsity of biosignals. Specifically, (i) tiny machine learning models to eliminate irrelevant biosignal patterns is developed, (ii) compressive sampling of relevant biosignals with appropriate sparse wavelet domains is efficiently performed, and (iii) hardware and OS operations to push processing efficiency is optimized. PROS also provides an abstraction layer, so the application only needs to care about detected relevant bio signal patterns without knowing the optimizations underneath. PROS is implemented and evaluated on two open biosignal datasets with 120 subjects and six biosignal patterns. The experimental results on unknown subjects of a practical use case such as epileptic seizure monitoring are very encouraging. PROS can reduce the streaming data rate by 24X while maintaining high fidelity signal. It boosts the power efficiency of the wearable device by more than 1200% and enables the ability to react to critical events immediately on the device. The memory and runtime overheads of PROS are minimal, with a few KBs and 10s of milliseconds for each biosignal pattern, respectively. PROS is currently adopted in research projects in multiple universities and hospitals. (Abstract). Document US 2023/334330 A1 (D2) discloses State of art techniques existing method refer to handling multiple objectives such as accuracy and latency. However, the reward functions are static and not tunable at user end. Further, for NN search with hardware constraints, approaches combine various techniques such as Reinforcement learning, Evolutionary Algorithm (EA) etc., however hardly any work attempts to disclose combining different NAS approaches in unison to reduce the search space of other. Embodiments of the present disclosure provide a method and system for automated creation of tiny Deep Learning (DL) models to be deployed on a platform having a set of hardware constraints. The method performs a coarse-grained search using a Fast EA NAS model and then utilizes a fine-grained search to identify customized and optimized tiny model. The coarse-grained search and the fine-grained search performed by agents based on a weighted multi-objective reward function, which are tunable at user end (Abstract).

SUMMARY

Embodiments of the present disclosure present technological improvements as solutions to one or more of the above-mentioned technical problems recognized by the inventors in conventional systems. The invention is set out in appended set of claims.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1A is a functional block diagram of a system, for generating tiny Deep Q-Network (DQN) models with optimal set of sensors for wearable device-based pain assessment, in accordance with some embodiments of the present disclosure.

FIG. 1B illustrates an architectural overview of the system of FIG. 1A, in accordance with some embodiments of the present disclosure.

FIG. 2 is a flow diagram illustrating a method for generating tiny Deep Q-Network (DQN) models with optimal set of sensors for wearable device-based pain assessment, using the system depicted in FIG. 1A and 1B, in accordance with some embodiments of the present disclosure.

FIG. 3 depicts generating a tiny DQN model for pain assessment with a sensor factor inclusive objective function and a Neural Network (NN) architecture search action space that includes number of sensors and sensor combination selected from among the set of on device sensors of the wearable device, in accordance with some embodiments of the present disclosure.

It should be appreciated by those skilled in the art that any block diagrams herein represent conceptual views of illustrative systems and devices embodying the principles of the present subject matter. Similarly, it will be appreciated that any flow charts, flow diagrams, and the like represent various processes which may be substantially represented in computer readable medium and so executed by a computer or processor, whether or not such computer or processor is explicitly shown.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible; the invention is defined by the scope of the appended claims.

Affective patterns in pain assessment refer to the subjective component of perception of pain, in addition to the behavior of physiological components and are dependent on a number of factors other than the objective features.. Features extracted from these affective patterns are learnt by Machine Learning (ML) models to predict pain. While the effect of these affective patterns is seen in some physiological signals, the cause and origin of pain can be detected from other sensor inputs. Thus, the features and indications extracted and the signature due to manifestation of each form of pain changes, resulting in change of the optimal model architecture. Hence, it is required to explore multi-sensor detection of pain from wearable devices. For example, the 'WellAff™' system aims to recognize affective states for wellbeing support. There are similar works that include health care scenarios along with wellbeing, especially the affective state and mental wellbeing of people with chronic diseases.

There is a need for large-scale study in the field and multiple off-the-shelf devices available for collection of data related to the detection of physical activity, sleep, stress, emotion, and analyzing the same. The types of sensors and quality of raw signals are important factors in the same. There is no versatile device suitable for all these purposes at once. Commonly used devices are Empatica E4™ and Samsung Galaxy™ Watch, for physiological signals to design ML classifier to recognize affective states. There has been some research on the association of pain with other affective states such as stress and emotions. The study of pain itself comes with certain roadblocks, the first being availability of data. It is difficult to design pain-related experiments without raising ethical concerns. There are existing pain-inducing tests that target studying the relation between pre-existing pain conditions and specific postures and movements such as different sitting and supine postures to study the effects on existing back-pain. A lot of pain-related research is done by observing recovering patients in the medical settings or in the process of surgery. The devices used in these scenarios are generally viable in hospital settings, in surgery rooms and are often expensive.

The study of pain and its relationship with stress via affective computing on wearables through suitable experimental settings is not fully explored and is necessary to make pan assessment process more ubiquitous and affordable.

In current State-of-the-Art (SoA), none of the research works have been found to focus on real-time detection of pain using various sensor features either manually or using auto-generated approach through deep neural networks, and design of feedback system for adjusting pain medication dosage along with other techniques to address or inhibit the sensation of pain. Although there is extensive work on stress detection and Affective Wearables utilizing multiple sensors, there is no similar approach of utilizing multi-sensor fusion for pain detection and design of a pervasive system to select sensors in an intelligent manner, to detect and quantify the pain level in an effective way, on edge devices, without interference or intervention of any person.

There is a wide range of applications, scenarios, and sensing modalities in the domain of affective computing and pain detection from wearable sensors. Depending on the requirement and type of pain to be detected, and whether pain level is to be computed, the various parameters and metrics vary. This is not feasible if every model is to be designed and optimized manually. The transfer between the different types of requirements can be accelerated and made scalable with the help of an automation framework. Sensing modalities - EEG, EDA, EMG, and ECG, with the addition of IMU and PPG signals (commonly available on wearable devices), are not always present on commercially available wearable devices.

The study of Physiology and Pathway of pain provides a theoretical domain background for the design of the whole system. The changes in features and the best working sensors for a given pain depends on the origin and pathway of pain and reflects in the physiological signals.

Embodiments of the present disclosure provide a method and system for generating tiny Deep Q-Network (DQN) models with the optimal set of sensors for wearable device-based pain assessment. The system provides an automation framework to auto-generate customized pain detection models as per requirement and based on the availability of 'on device sensors' and the device constraints. Thus, the system is scalable for multi-device application (different types of wearable devices equipped with different combination of sensors) and can provide the flexibility required to adapt to the use-case settings.

The system enables real-time detection of pain from multi-modal sensor signal inputs from a wearable device using domain knowledge-guided feature generation. An optimal set of unique sensor combinations is identified available on the wearable device for a pain type of interest. A closed feedback mechanism is provided for real-time response to the detected pain episodes in the form of a pain-relief mechanism (pain medication dosage, stimulation, or verbal feedback).

The system provides an intelligent sensor selection and fusion mechanism to detect the presence of pain and quantify pain level on edge devices in an effective and efficient manner, guided by information on pain origin, pathway and type extracted from input signals, without the requirement of any manual intervention or interference. The system provides an automation framework to accelerate the deployment of customized tiny models for wearable and edge devices, based on sensor availability and device compute capacity to create suitable model for the target device and task. The system takes accuracy, model size and latency as objectives, and sensor availability and computation resource constraints as targets for the automation framework. The automation technique enables rapid generation and deployment of models on multiple edge devices (wearable devices), independent of the availability of a specific sensor. The multi-objective optimization technique would result in highly optimized models, suitable for real-time scenarios, and advantageous for deployment on devices with higher computation power as well, since in continuous monitoring scenarios, minimizing the power consumption always serves as a necessary benefit of the complete system.

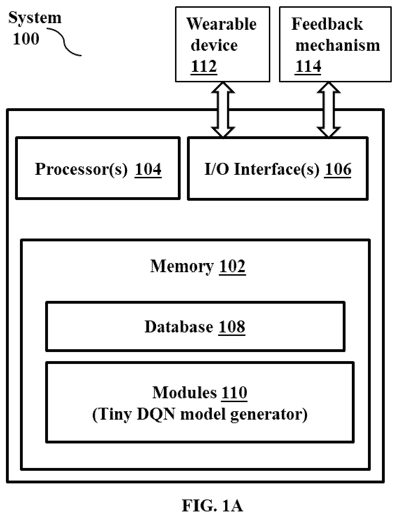

Referring now to the drawings, and more particularly to FIGS. 1A through 3, where similar reference characters denote corresponding features consistently throughout the figures, there are shown preferred embodiments, and these embodiments are described in the context of the following exemplary system and/or method.

FIG. 1A is a functional block diagram of a system 100 for generating tiny Deep Q-Network (DQN) models with optimal set of sensors for wearable device-based pain assessment, in accordance with some embodiments of the present disclosure.

In an embodiment, the system 100 includes a processor(s) 104, communication interface device(s), alternatively referred as input/output (I/O) interface(s) 106, and one or more data storage devices or a memory 102 operatively coupled to the processor(s) 104. The system 100 with one or more hardware processors is configured to execute functions of one or more functional blocks of the system 100.

Referring to the components of system 100, in an embodiment, the processor(s) 104, can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, and the like.

The I/O interface(s) 106 can include a variety of software and hardware interfaces, for example, a web interface, a graphical user interface and the like and can facilitate multiple communications with a plurality of wearable devices such as wearable device 112 and feedback mechanism 114 associated with the wearable device 112. The communication with coupled devices, external system can be using a wide variety of networks N/W and protocol types, including wired networks, for example, LAN, cable, etc., and wireless networks, such as WLAN, cellular and the like. In an embodiment, the I/O interface (s) 106 can include one or more ports for connecting to a number of external devices or to another server or devices.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes.

In an embodiment, the memory 102 includes a plurality of modules 110 such as Tiny DQN model generator for generation tiny DQN models to be deployed on the wearable device 112 for pain assessment. Further, the plurality of modules 110 include programs or coded instructions that supplement applications or functions performed by the system 100 for executing different steps involved in the process of generating tiny Deep Q-Network (DQN) models with optimal set of sensors for wearable device-based pain assessment, being performed by the system 100. The plurality of modules 110, amongst other things, can include routines, programs, objects, components, and data structures, which performs particular tasks or implement particular abstract data types. The plurality of modules 110 may also be used as, signal processor(s), node machine(s), logic circuitries, and/or any other device or component that manipulates signals based on operational instructions. Further, the plurality of modules 110 can be used by hardware, by computer-readable instructions executed by the one or more hardware processors 104, or by a combination thereof. The plurality of modules 110 can include various sub-modules (not shown).

Further, the memory 102 may comprise information pertaining to input(s)/output(s) of each step performed by the processor(s) 104 of the system 100 and methods of the present disclosure. Further, the memory 102 includes a database 108. The database (or repository) 108 may include the generated tiny DQN models for each of the plurality of wearable devices such as tiny DQN model for wearable device112). Further the database 108 may include a plurality of abstracted pieces of code for refinement and data that is processed, received, or generated as a result of the execution of the plurality of modules in the module(s) 110.

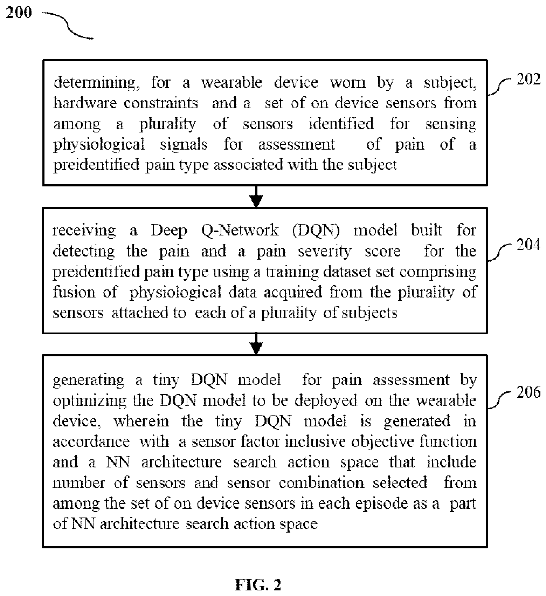

Although the data base 108 is shown internal to the system 100, it will be noted that, in alternate embodiments, the database 108 can also be implemented external to the system 100, and communicatively coupled to the system 100. The data contained within such an external database may be periodically updated. For example, new data may be added into the database (not shown in FIG. 1A) and/or existing data may be modified and/or non-useful data may be deleted from the database. In one example, the data may be stored in an external system, such as a Lightweight Directory Access Protocol (LDAP) directory and a Relational Database Management System (RDBMS). Functions of the components of the system 100 are now explained with reference to steps in flow diagrams in FIG. 2 through FIG. 3.

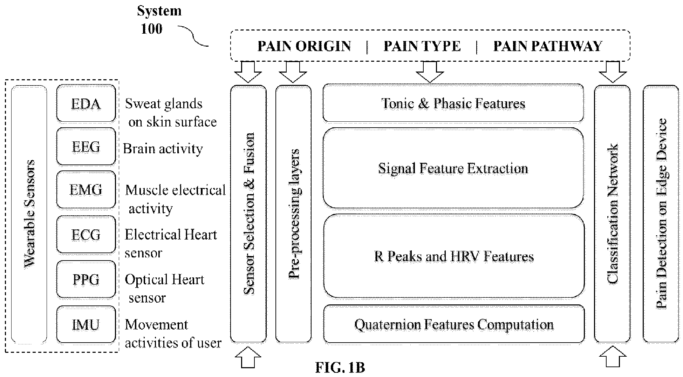

FIG. 1B illustrates an architectural overview of the system 100 of FIG. 1A, in accordance with some embodiments of the present disclosure. As depicted, the system 100 addresses multiple sensors available in commercially available wearable devices. In example implementation the sensors defined in the system 100 are Electrodermal Activity (EDA), Electrocardiography (ECG). Photoplethysmography (PPG), Electroencephalography, (EEG), Electromyography (EMG), and Inertial Measurement Unit (IMU) sensors. Each of these sensors have different features to be extracted for the detection of pain episodes. Also, the frequency and mode of sensing (unobtrusive and continuous or periodic monitoring) and availability of sensors are important factors to be addressed, to make the system robust, and suitable for a wide variety of wearable devices. The EDA signals have been widely utilized for Affective Computing applications, since it reflects skin conductance responses owing to sweat gland activity as a result some underlying sympathetic reaction to an applied stimulus. This covers a number of activities and events, including perception of pain. Since EDA is an unobtrusive mechanism and is commonly available in a wearable form factor, it is preferred for detecting physiological events such as the occurrence of pain. Apart from EDA, signals, which are also commonly available on wearable devices, ECG, PPG and IMU - can be used to measure Heart Rate Variability (HRV) and motion constraints of a user. HRV and mobility features have also been used to detect pain in previous work. Apart from these, EEG and EMG signals provide features that can be utilized to detect pain-related events in a user. However, these sensors may require a more controlled environment for measurement.

The system 100 is designed in such a way that the individual signals are processed, and features extracted with the use of domain knowledge, information on the origin, pathway and type of pain, and the fusion of two or more sensor data are employed to improve the performance obtained from a single sensor. The automation technique enables efficient and quick generation of customized models (tiny DQN models by optimizing a Deep Q-Network (DQN) model built for detecting presence/absence of pain and a pain severity score for the preidentified pain type for any target device (wearable device 112), given the inputs of device constraints and available sensors. This removes the constraint that the pain detection system is dependent on the availability of a particular sensor on a selected wearable device.

**Physiology and Pathway of Pain:** Pain is an important phenomenon of the nervous system which provides the body with a warning of potential or actual injury. The experience of pain involves both sensory and emotional components, affected by several factors including psychological factors in addition to various psychological processes. The transmission of pain involves several complex processes starting from the nociceptive receptors all over the body, to the thalamus in the brain. The origin of pain can be Somatic, Visceral, Neuropathic, or psychological. Depending on the origin, the pathway of pain, as well as the inhibitory measures vary. The basic mechanism of pain involves three events-transduction, transmission and modulation in case of noxious or unpleasant stimuli such as in case of nociceptive pathway, the transduction occurs in the order as follows: First, the conversion of stimulus events to chemical tissue events, then the conversion of chemical tissue and synaptic cleft events into electrical events in neurons and finally the transduction of electrical events in the neurons in the form of chemical events at the synapses. Post completion of the transduction process, the transmission of the electrical events takes place along the neuron pathways, while information is transmitted through cells by neurotransmitters in the synaptic cleft from a post-synaptic terminal of one cell to a pre-synaptic terminal of another. The modulation process occurs at all levels of nociceptive pathways through the primary afferent neuron, dorsal horn of the spinal cord and higher brain centre by up or down-regulation. These steps lead to the initiation and completion of the pathway of pain, resulting in the feeling or experience of the painful sensation triggered by some stimulus.

As described above, like the nociceptive pathway, there are various origins and stimuli resulting in pain, which also follow different pathways. It would be helpful to obtain information about the origin and pathway of pain from the collected Physiological signals. In currently available research work, and available datasets, the pain is generally simulated through external stimuli such as heat, and the source is fixed. Thus, given a user with some form of pain, it would be useful to find the origin of pain in real-world applications. Also, the distinction between acute pain and chronic pain is not studied in AI-based solutions. A detailed study of the above parameters and understanding the impact of pain pathway from wearable and physiological signals is a significant area addressed by the system and method disclosed herein..

FIG. 2 is a flow diagram illustrating a method 200 for generating tiny Deep Q-Network (DQN) models with optimal set of sensors for wearable device-based pain assessment, using the system depicted in FIG. 1A and 1B, in accordance with some embodiments of the present disclosure.

In an embodiment, the system 100 comprises one or more data storage devices or the memory 102 operatively coupled to the processor(s) 104 and is configured to store instructions for execution of steps of the method 200 by the processor(s) or one or more hardware processors 104. The steps of the method 200 of the present disclosure will now be explained with reference to the components or blocks of the system 100 as depicted in FIG. 1A and 1B and the steps of flow diagram as depicted in FIG. 2. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

A scenario considered is for pain assessment of a subject via the wearable device 112 worn by the subject. The wearable device 112 is equipped with a limited specific set of on device sensors (few from among the plurality of sensors comprising Electro-dermal Activity (EDA), Electrocardiography (ECG). Photoplethysmography (PPG), Electroencephalography, (EEG), Electromyography (EMG), and Inertial Measurement Unit (IMU) sensors that system 100 is designed for.

Referring to the steps of the method 200, at step 202 of the method 200, the one or more hardware processors 104 are configured by the instructions to determine, for the wearable device 112 worn by the subject, hardware constraints and the set of on device sensors for sensing physiological signals for assessment of pain of a preidentified pain type associated with the subject.

At step 204 of the method 200, the one or more hardware processors 104 are configured by the instructions to receive the trained DQN model built for detecting the pain and the pain severity score for the preidentified pain type using a training dataset set comprising fusion of physiological data acquired from the plurality of sensors attached to each of a plurality of subjects.

The DQN model, also referred to as large DQN model, is pretrained for the predefined pain type (pain type of interest) is built using wide range of sensors available on wearable devices.

At step 206 of the method 200, the one or more hardware processors 104 are configured by the instructions to generate the tiny DQN model for pain assessment by optimizing the DQN model to be deployed on the wearable device 112. The tiny DQN model is generated in accordance with a sensor factor inclusive objective function $O_m$ and a Neural Network (NN) architecture search action space that includes a number of sensors $(N_m)$ and sensor combination selected from among the set of on device sensors in each episode as a part of the NN architecture search action space.

The sensor factor inclusive objective function enables selecting an optimal number of on device sensors and a unique combination of sensors which is a subset of all on-device sensors for the preidentified pain type along with the hardware constraints from the NN architecture search action space. The sensor factor inclusive objective function is based on number of sensors selected in each episode, and a reward function defined as a function of weighted performance metrics $P=\{$ Accuracy : $a_m$, Model Size: $s_m$, Peak Memory : $m_m$, and Multiply-Accumulate : $mac_m$.

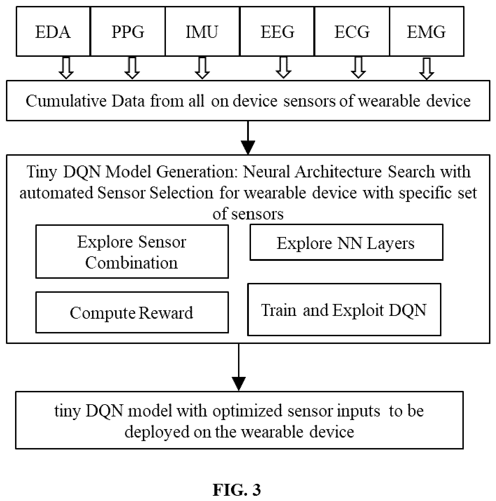

**Generating tiny DQN model:** As depicted in FIG. 3, the data acquisition includes collecting physiological signal data from multiple wearable sensors and edge device and storing in a signal processing buffer. Extraction of features related to pain is performed, wherein the raw signals are pre-processed by standard techniques, such as baseline removal, normalization, filtering, and noise removal. Following this, Time domain and Spectral features relevant to pain (pain type for which the tiny DQN model is to be generated trained) are extracted from each of the signals, to be processed further with the help of ML/DL techniques. Fusion of sensor data is performed by extracting the pain-specific (pain type specific) features from the different signals and merging them. Thereafter the features in combination with the raw signals are fed to the pretrained DQN model to learn the existence and intensity of pain. The sensor data fusion method can be enhanced with the help of AutoML or Neural Architecture Search techniques.

**Sensor factor inclusive objective function for a candidate model m (tiny DQN model)** : It can be noted that number of sensors $N_m$ determines overall power required by the tiny DQN model

Input metrics :

    1. No. of sensors : $N_m$
    2. Value of sensors : $Ssub_m$ = subset of $\{S_1, S_2, ...., S_{ns,m}\}$, $(S_i, 1 <= i <= N_m)$
    3. Accuracy : $a_m$
    4. Model Size : $s_m$
    5. Peak Memory : $m_m$
    6. Multiply-Accumulate : $mac_m$

Given a set of *performance metrics* $P = \{a_m, s_m, m_m, mac_m\}$, (where P is not limited to 4 values, and can be a set of any N values as per task/application/ user choice) and a set of corresponding priority weights w = {$w_1, w_2, w_3, w_4$}, (where $w_i$ can be N values depending on the length of set P).

$$Reward\ R_m = R(a_m, s_m, m_m, mac_m) \text{ -- } (1)\ R_m = sum[w_i * f(P_i)] \text{ -- } (2)$$

where $f(P_i)$ is a linear or exponential expression of the metric, depending on the objective of the corresponding metric and the task in hand. The Objective function

$$O_m = g(N_m)\ *R_m \text{ -- } (3)$$

where g(Nm) is an expression inversely proportional to the number of sensors,

$$g(N_m) = 1/(N_m)^k \text{ -- } (4)$$

where k is based on the criticality of the device power. As a default value, k = 0.05, where the Reward Rm is attenuated by a coefficient of 0.96 when the number of sensors $N_m$ = 2.

A unique Sensor combination ID corresponding to subset Ssubm denoting the exact combination of sensors selected, is a part of Architecture search Action space given by:

$$A = [Ssub_m, layer\_index, layer\_type, kernel\_size, stride, n\_channels, termination] \text{ -- } (5)$$

For layer_index = 0, since number of sensors and combination are selected at the start of an episode. Each term in equation (5) corresponds to the configuration of a selected layer. The sensor selection factors into the final objective function in two different ways - a. direct and b. indirect. The direct form is as given in equation (3) where the sensor coefficient factors into the objective function, and the indirect form is through equation (1) where the metrics $s_m$, $m_m$, and $mac_m$ are affected by the number of sensors $N_m$, since no. of input features Nr is directly proportional to Nm and model size, multiply-accumulate operations and memory usage are directly proportional to the Number of Features $N_f$.

Thus, herein the NN architecture sampling has varying no. of sensors during NAS exploration phase. The sensors can be kept as a variable parameter, similar to the no. of layers in the model architecture.

Once the tiny DQN model is generated, the tiny DQN model is deployed on the wearable device 112 worn by the subject for real time inferencing. The inferencing can be either only detecting absence or presence of the pain or can be also providing the pain severity score. The steps during real time inferencing include:

a. Receiving the physiological signals captured by the optimal set of sensors.
b. Preprocessing the received physiological signals:
c. Obtaining a set of time domain pain features and a set of spectral pain features from the pre-processed physiological signals; and
d. Predicting one of the absence or presence of the pain and the pain severity score for the subject.

Further, the system 100 integrates the inference into the feedback mechanism 114 providing pain relief to the subject. A first feedback mechanism is provided for adjusting a pain medication dosage delivered to the subject in accordance with the pain severity score and generating an alert notification shared to a device of a clinical administrator if the pain severity score is above a predefined pain threshold, wherein the pain medication dosage is computed in accordance with the equation 6 below:

$$Pain\ medication\ dosage = px\_n * [total\_dosage] \quad \text{----}(6),$$

wherein , $px\_n$ is a Normalized Pain Score computed based on the pain severity score, the maximum and minimum value set for the pain severity score.

The first feedback mechanism may be implemented with a pain medication infusion pump which provides the correct dosage of medication to the subject depending on the level of pain detected.

The system 100 also integrates the inferencing to a second feedback mechanism for actuating a pain relief stimulation signal or a verbal relief to the subject. The pain relief in accordance with the normalized pain severity score is based on equation 7 below:

**EP 4 563 078 B1**

$$Actuated\ pain\ relief\ stimulation\ signal = px\_n * [input\_voltage] \qquad (7)$$

The pain relief technique may include a motor-based nerve stimulation device, which supplies an input voltage based on the detected pain level to the motor input terminals, and a stimulation signal is provided to the user, where the strength of the stimulation signal is directly proportional to the input voltage

The written description describes the subject matter herein to enable any person skilled in the art to make and use the embodiments. The scope of the invention is defined by the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (200) for pain assessment, the method comprising:

    determining (202) by one or more hardware processors, for a wearable device worn by a subject, hardware constraints and a set of on device sensors from among a plurality of sensors identified for sensing physiological signals for assessment of pain of a preidentified pain type associated with the subject,
    receiving (204) by one or more hardware processors, a Deep Q-Network (DQN) model built for detecting the pain and a pain severity score for the preidentified pain type, wherein the DQN model is trained using a training dataset set comprising fusion of physiological data acquired from the plurality of sensors attached to each of a plurality of subjects;

generating (206) by one or more hardware processors, a tiny DQN model for pain assessment to be deployed on the wearable device by optimizing the DQN model, wherein the tiny DQN model is generated in accordance with a sensor factor inclusive objective function $(O_m)$ and a Neural Network (NN) architecture search action space specifies a number of sensors $(N_m)$ and sensor combination selected from among the plurality of on device sensors in each episode as a part of the NN architecture search action space,

wherein the sensor factor inclusive objective function enables selecting an optimal number of on device sensors and a unique sensor combination which is a subset of the set of on device sensors for the preidentified pain type along with the hardware constraints from the NN architecture search action space, and

wherein the sensor factor inclusive objective function is based on the number of sensors $(N_m)$ selected in each episode, and a reward function $(R_m)$ defined as a function of weighted performance metrics comprising, $P=$ { *Accuracy : $a_m$, Model Size : $s_m$, Peak Memory : $m_m$, and Multiply-Accumulate : $mac_m$,* wherein the sensor factor inclusive objective function is mathematically represented as $O_m = g(N_m) *R_m$, where $g(N_m)$ is an expression inversely proportional to the number of sensors, $g(N_m) = 1/(N_m)^k$, k is based on the criticality of the device power, and $R_m$ is the reward function.

2. The method as claimed in claim 1, wherein the generated tiny DQN model is deployed on the wearable device worn by the subject for real time inferencing of one of i) absence or presence of the pain and ii) the pain severity score, the real time inferencing comprising:

receiving the physiological signals captured by the optimal set of sensors;
preprocessing the received physiological signals:

obtaining a set of time domain pain features and a set of spectral pain features from the pre-processed physiological signals; and
predicting one of the absence or presence of the pain and the pain severity score for the subject.

3. A system (100) for pain assessment, the system (100) comprising:

a memory (102) storing instructions;
one or more Input/Output (I/O) interfaces (106); and
one or more hardware processors (104) coupled to the memory (102) via the one or
more I/O interfaces (106), wherein the one or more hardware processors (104) are configured by the instructions to:

determine, for a wearable device (112) worn by a subject, hardware constraints and a set of on device sensors from among a plurality of sensors identified for sensing physiological signals for assessment of pain of a preidentified pain type associated with the subject,

receive a Deep Q-Network (DQN) model built for detecting the pain and a pain severity score for the preidentified pain type, wherein the DQN model is trained using a training dataset set comprising fusion of physiological data acquired from the plurality of sensors attached to each of a plurality of subjects; and
generate a tiny DQN model for pain assessment to be deployed on the wearable device by optimizing the DQN model, wherein the tiny DQN model is generated in accordance with a sensor factor inclusive objective function $(O_m)$ and a Neural Network (NN) architecture search action space that include a number of sensors $(N_m)$ and sensor combination selected from among the set of on device sensors in each episode as a part of the NN architecture search action space,

wherein the sensor factor inclusive objective function enables selecting an optimal number of on device sensors and a unique sensor combination which is a subset of the set of on device sensors for the preidentified pain type along with the hardware constraints from the NN architecture search action space, and

wherein the sensor factor inclusive objective function is based on number of sensors $(N_m)$ selected in each episode, and a reward function $(R_m)$ defined as a function of weighted performance metrics comprising $P=$ { *Accuracy $a_m$, Model Size : $s_m$, Peak Memory : $m_m$, and Multiply-Accumulate : $mac_m$,* wherein the sensor factor inclusive objective function is mathematically represented as $O_m = g(N_m) *R_m$, where $g(N_m)$ is an expression inversely proportional to the number of sensors, $g(N_m) = 1/(N_m)^k$, k is based on the criticality of the device power, and $R_m$ is the reward function.

4. The system as claimed in claim 3, wherein the one or more hardware processors (104) are configured to deploy the generated tiny DQN model on the wearable device (112)worn by the subject for real time inferencing of one of i) absence or presence of the pain and ii) the pain severity score by, wherein real time inferencing comprising:

receiving the physiological signals captured by the optimal set of sensors;
preprocessing the received physiological signals:

obtaining a set of time domain pain features and a set of spectral pain features from the pre-processed physiological signals; and
predicting one of the absence or presence of the pain and the pain severity score for the subject.

5. The system as claimed in claim 4, wherein the one or more hardware processors (104) are configured to trigger a first feedback mechanism for adjusting a pain medication dosage delivered to the subject in accordance with the pain severity score and generating an alert notification shared to a device of a clinical administrator if the pain severity score is above a predefined pain threshold, wherein the pain medication dosage is computed in accordance with the equation:
*Pain medication dosage = px_n * [total_dosage]*, wherein , *px_n* is a Normalized Pain Score computed based on the pain severity score, the maximum and minimum value set for the pain severity score.

6. The system as claimed in claim 4, wherein the one or more hardware processors (104) are configured to trigger a second feedback mechanism for actuating a pain relief stimulation signal or a verbal relief to the subject, wherein the pain relief stimulation signal is in accordance with the normalized pain severity score based on equation *Actuated pain relief stimulation signal = px_n * [input_voltage]*.

7. One or more non-transitory machine readable information storage mediums comprising one or more instructions which when executed by one or more hardware processors cause:

determining for a wearable device worn by a subject, hardware constraints and a set of on device sensors from among a plurality of sensors identified for sensing physiological signals for assessment of pain of a preidentified pain type associated with the subject,
receiving a Deep Q-Network (DQN) model built for detecting the pain and a pain severity score for the preidentified pain type, wherein the DQN model is trained using a training dataset set comprising fusion of physiological data acquired from the plurality of sensors attached to each of a plurality of subjects;
generating a tiny DQN model for pain assessment to be deployed on the wearable device by optimizing the DQN model, wherein the tiny DQN model is generated in accordance with a sensor factor inclusive objective function $(O_m)$ and a Neural Network (NN) architecture search action space specifies a number of sensors $(N_m)$ and sensor combination selected from among the plurality of on device sensors in each episode as a part of the NN architecture search action space,
wherein the sensor factor inclusive objective function enables selecting an optimal number of on device sensors and a unique sensor combination which is a subset of the set of on device sensors for the preidentified pain type along with the hardware constraints from the NN architecture search action space, and
wherein the sensor factor inclusive objective function is based on the number of sensors $(N_m)$selected in each episode, and a reward function $(R_m)$defined as a function of weighted performance metrics comprising, *P= {Accuracy : $a_m$, Model Size : $s_m$, Peak Memory : $m_m$, and Multiply-Accumulate : $mac_m$*, wherein the sensor factor inclusive objective function is mathematically represented as $O_m = g(N_m) *R_m$, where $g(N_m)$ is an expression inversely proportional to the number of sensors, $g(N_m) = 1/(N_m)^k$, k is based on the criticality of the device power, and $R_m$
is the reward function.

8. The one or more non-transitory machine-readable information storage mediums as claimed in claim 7, wherein the generated tiny DQN model is deployed on the wearable device worn by the subject for real time inferencing of one of i) absence or presence of the pain and ii) the pain severity score, the real time inferencing comprising:

receiving the physiological signals captured by the optimal set of sensors;
preprocessing the received physiological signals:

obtaining a set of time domain pain features and a set of spectral pain features from the pre-processed physiological signals; and

predicting one of the absence or presence of the pain and the pain severity score for the subject.

9. The one or more non-transitory machine-readable information storage mediums as claimed in claim 8, comprising triggering a first feedback mechanism for adjusting a pain medication dosage delivered to the subject in accordance with the pain severity score and generating an alert notification shared to a device of a clinical administrator if the pain severity score is above a predefined pain threshold, wherein the pain medication dosage is computed in accordance with the equation:
Pain medication dosage = px_n * [total_dosage], wherein px_n is a Normalized Pain Score computed based on the pain severity score, the maximum and minimum value set for the pain severity score.

10. The one or more non-transitory machine-readable information storage mediums as claimed in claim 9, comprising triggering a second feedback mechanism for actuating a pain relief stimulation signal or a verbal relief to the subject, wherein the pain relief stimulation signal is in accordance with the normalized pain severity score based on equation

$$Actuated\ pain\ relief\ stimulation\ signal = px\_n * [input\_voltage]$$

**Patentansprüche**

1. Prozessorimplementiertes Verfahren (200) zur Schmerzbeurteilung, wobei das Verfahren Folgendes umfasst:

Bestimmen (202), durch einen oder mehrere Hardwareprozessoren, für eine tragbare Vorrichtung, die von einem Subjekt getragen wird, von Hardwarebeschränkungen und einem Satz von Sensoren an der Vorrichtung aus einer Mehrzahl von Sensoren, die zum Erfassen physiologischer Signale zur Beurteilung von Schmerzen eines voridentifizierten, mit dem Subjekt assoziierten Schmerztyps identifiziert werden,
Empfangen (204), durch einen oder mehrere Hardwareprozessoren, eines Deep-Q-Network(DQN)-Modells, das zum Erkennen der Schmerzen und eines Schmerzschwerewerts für den voridentifizierten Schmerztyp erstellt wurde, wobei das DQN-Modell unter Verwendung eines Trainingsdatensatzsatzes trainiert wird, der eine Fusion physiologischer Daten umfasst, die von der Mehrzahl von Sensoren erfasst werden, die an jedem einer Mehrzahl von Subjekten angebracht sind;
Erzeugen (206), durch einen oder mehrere Hardwareprozessoren, eines winzigen DQN-Modells zur Schmerzbeurteilung, das auf der tragbaren Vorrichtung durch Optimieren des DQN-Modells eingesetzt werden soll, wobei das winzige DQN-Modell gemäß einer Sensorfaktor-Einschluss-Zielfunktion $(O_m)$ erzeugt wird und ein Neural-Network(NN)-Architektur-Suchaktionsraum eine Anzahl von Sensoren $(N_m)$ und eine Sensorkombination, die aus der Mehrzahl von Sensoren an der Vorrichtung ausgewählt wird, in jeder Episode als Teil des NN-Architektur-Suchaktionsraums spezifiziert,
wobei die Sensorfaktor-Einschluss-Zielfunktion das Auswählen einer optimalen Anzahl von Sensoren an der Vorrichtung und einer eindeutigen Sensorkombination, die eine Teilmenge des Satzes von Sensoren an der Vorrichtung für den voridentifizierten Schmerztyp ist, zusammen mit den Hardwarebeschränkungen aus dem NN-Architektur-Suchaktionsraum ermöglicht, und
wobei die Sensorfaktor-Einschluss-Zielfunktion auf der Anzahl von Sensoren $(N_m)$, die in jeder Episode ausgewählt werden, und einer Belohnungsfunktion $(R_m)$ basiert, die als eine Funktion von gewichteten Leistungsmetriken definiert ist, die P={Genauigkeit : $a_m$, Modellgröße : $s_m$, Spitzenspeicher : $m_m$ und Multiplikativakkumulieren : $mac_m$ umfassen, wobei die Sensorfaktor-Einschluss-Zielfunktion mathematisch als $O_m = g(N_m) * R_m$ dargestellt wird, wobei $g(N_m)$ ein Ausdruck ist, der umgekehrt proportional zur Anzahl von Sensoren ist, $g(N_m) = 1/(N_m)^k$ , k auf der Kritikalität der Vorrichtungsleistung basiert und $R_m$ die Belohnungsfunktion ist.

2. Verfahren nach Anspruch 1, wobei das erzeugte winzige DQN-Modell auf der tragbaren Vorrichtung, die von dem Subjekt getragen wird, zur Echtzeit-Schlussfolgerung von einem von i) Abwesenheit oder Vorhandensein der Schmerzen und ii) dem Schmerzschwerewert eingesetzt wird, wobei die Echtzeit-Schlussfolgerung Folgendes umfasst:

Empfangen der physiologischen Signale, die von dem optimalen Satz von Sensoren erfasst werden;
Vorverarbeiten der empfangenen physiologischen Signale:

Erhalten eines Satzes von Zeitdomänen-Schmerzmerkmalen und eines Satzes von spektralen Schmerzmerkmalen aus den vorverarbeiteten physiologischen Signalen; und

Vorhersagen von einem von der Abwesenheit oder dem Vorhandensein der Schmerzen und dem Schmerzschwerewert für das Subjekt.

3. System (100) zur Schmerzbeurteilung, wobei das System (100) Folgendes umfasst:

einen Speicher (102), der Anweisungen speichert;
eine oder mehrere Eingabe/Ausgabe(E/A)-Schnittstellen (106); und
einen oder mehrere Hardwareprozessoren (104), die über die eine oder die mehreren E/A-Schnittstellen (106) mit dem Speicher (102) gekoppelt sind, wobei der eine oder die mehreren Hardwareprozessoren (104) durch die Anweisungen konfiguriert sind zum:

Bestimmen, für eine tragbare Vorrichtung (112), die von einem Subjekt getragen wird, von Hardwarebeschränkungen und einem Satz von Sensoren an der Vorrichtung aus einer Mehrzahl von Sensoren, die zum Erfassen physiologischer Signale zur Beurteilung von Schmerzen eines voridentifizierten, mit dem Subjekt assoziierten Schmerztyps identifiziert werden,
Empfangen eines Deep-Q-Network(DQN)-Modells, das zum Erkennen der Schmerzen und eines Schmerzschwerewerts für den voridentifizierten Schmerztyp erstellt wurde, wobei das DQN-Modell unter Verwen-dung eines Trainingsdatensatzes trainiert wird, der eine Fusion physiologischer Daten umfasst, die von der Mehrzahl von Sensoren erfasst werden, die an jedem einer Mehrzahl von Subjekten angebracht sind; und
Erzeugen eines winzigen DQN-Modells zur Schmerzbeurteilung, das auf der tragbaren Vorrichtung durch Optimieren des DQN-Modells eingesetzt werden soll, wobei das winzige DQN-Modell gemäß einer Sen-sorfaktor-Einschluss-Zielfunktion ($O_m$) und einem Neural-Network(NN)-Architektur-Suchaktionsraum erzeugt wird, die eine Anzahl von Sensoren ($N_m$) und eine Sensorkombination, die aus dem Satz von Sensoren an der Vorrichtung ausgewählt wird, in jeder Episode als Teil des NN-Architektur-Suchaktionsraums beinhalten,
wobei die Sensorfaktor-Einschluss-Zielfunktion das Auswählen einer optimalen Anzahl von Sensoren an der Vorrichtung und einer eindeutigen Sensorkombination, die eine Teilmenge des Satzes von Sensoren an der Vorrichtung für den voridentifizierten Schmerztyp ist, zusammen mit den Hardwarebeschränkungen aus dem NN-Architektur-Suchaktionsraum ermöglicht, und
wobei die Sensorfaktor-Einschluss-Zielfunktion auf der Anzahl von Sensoren ($N_m$), die in jeder Episode ausgewählt werden, und einer Belohnungsfunktion ($R_m$) basiert, die als eine Funktion von gewichteten Leistungsmetriken definiert ist, die P={Genauigkeit : $a_m$, Modellgröße : $s_m$, Spitzenspeicher : $m_m$ und Multiplikativ-akkumulieren : $mac_m$ umfassen, wobei die Sensorfaktor-Einschluss-Zielfunktion mathematisch als $O_m = g(N_m) * R_m$ dargestellt wird, wobei $g(N_m)$ ein Ausdruck ist, der umgekehrt proportional zur Anzahl von Sensoren ist, $g(N_m) = 1/(N_m)^k$ , k auf der Kritikalität der Vorrichtungsleistung basiert und Rm die Belohnungsfunktion ist.

4. System nach Anspruch 3, wobei der eine oder die mehreren Hardwareprozessoren (104) konfiguriert sind, um das erzeugte winzige DQN-Modell auf der tragbaren Vorrichtung (112), die von dem Subjekt getragen wird, zur Echtzeit-Schlussfolgerung von einem von i) Abwesenheit oder Vorhandensein der Schmerzen und ii) dem Schmerzschwerewert einzusetzen, wobei die Echtzeit-Schlussfolgerung Folgendes umfasst:

Empfangen der physiologischen Signale, die von dem optimalen Satz von Sensoren erfasst werden;
Vorverarbeiten der empfangenen physiologischen Signale:

Erhalten eines Satzes von Zeitdomänen-Schmerzmerkmalen und eines Satzes von spektralen Schmerzmerkmalen aus den vorverarbeiteten physiologischen Signalen; und
Vorhersagen von einem von der Abwesenheit oder dem Vorhandensein der Schmerzen und dem Schmerzschwerewert für das Subjekt.

5. System nach Anspruch 4, wobei der eine oder die mehreren Hardwareprozessoren (104) konfiguriert sind, um einen ersten Rückkopplungsmechanismus zum Anpassen einer Schmerzmedikationsdosierung, die an das Subjekt abgegeben wird, gemäß dem Schmerzschwerewert auszulösen und eine Alarmbenachrichtigung zu erzeugen, die an eine Vorrichtung eines klinischen Administrators geteilt wird, wenn der Schmerzschwerewert über einem vordefinierten Schmerzschwellenwert liegt, wobei die Schmerzmedikationsdosierung gemäß der folgenden Gleichung berechnet wird:
Dosierung von Schmerzmitteln = px_n * [gesamt_Dosierung], wobei px_n ein normalisierter Schmerzwert ist, der basierend auf dem Schmerzschwerewert, dem maximalen und minimalen Wert, die für den Schmerzschwerewert eingestellt sind, berechnet wird.

6. System nach Anspruch 4, wobei der eine oder die mehreren Hardwareprozessoren (104) konfiguriert sind, um einen zweiten Rückkopplungsmechanismus zum Auslösen eines Schmerzlinderungsstimulationssignals oder einer verbalen Linderung für das Subjekt auszulösen, wobei das Schmerzlinderungsstimulationssignal gemäß dem normalisierten Schmerzschwerewert basierend auf der Gleichung

*Ausgelöstes Schmerzlinderungsstimulationssignal = px_n * [Eingabe_Spannung] ist.*

7. Ein oder mehrere nichtflüchtige maschinenlesbare Informationsspeichermedien, die eine oder mehrere Anweisungen umfassen, die bei Ausführung durch einen oder mehrere Hardwareprozessoren Folgendes bewirken:

Bestimmen, für eine tragbare Vorrichtung, die von einem Subjekt getragen wird, von Hardwarebeschränkungen und einem Satz von Sensoren an der Vorrichtung aus einer Mehrzahl von Sensoren, die zum Erfassen physiologischer Signale zur Beurteilung von Schmerzen eines voridentifizierten, mit dem Subjekt assoziierten Schmerztyps identifiziert werden,

Empfangen eines Deep-Q-Network(DQN)-Modells, das zum Erkennen der Schmerzen und eines Schmerzschwerewerts für den voridentifizierten Schmerztyp erstellt wurde, wobei das DQN-Modell unter Verwendung eines Trainingsdatensatzes trainiert wird, der eine Fusion physiologischer Daten umfasst, die von der Mehrzahl von Sensoren erfasst werden, die an jedem einer Mehrzahl von Subjekten angebracht sind;

Erzeugen eines winzigen DQN-Modells zur Schmerzbeurteilung, das auf der tragbaren Vorrichtung durch Optimieren des DQN-Modells eingesetzt werden soll, wobei das winzige DQN-Modell gemäß einer Sensorfaktor-Einschluss-Zielfunktion ($O_m$) erzeugt wird und ein Neural-Network(NN)-Architektur-Suchaktionsraum eine Anzahl von Sensoren ($N_m$) und eine Sensorkombination, die aus der Mehrzahl von Sensoren an der Vorrichtung ausgewählt wird, in jeder Episode als Teil des NN-Architektur-Suchaktionsraums spezifiziert,

wobei die Sensorfaktor-Einschluss-Zielfunktion das Auswählen einer optimalen Anzahl von Sensoren an der Vorrichtung und einer eindeutigen Sensorkombination, die eine Teilmenge des Satzes von Sensoren an der Vorrichtung für den voridentifizierten Schmerztyp ist, zusammen mit den Hardwarebeschränkungen aus dem NN-Architektur-Suchaktionsraum ermöglicht, und

wobei die Sensorfaktor-Einschluss-Zielfunktion auf der Anzahl von Sensoren ($N_m$), die in jeder Episode ausgewählt werden, und einer Belohnungsfunktion ($R_m$) basiert, die als eine Funktion von gewichteten Leistungsmetriken definiert ist, die *P={Genauigkeit : $a_m$, Modellgröße : $s_m$, Spitzenspeicher : $m_m$ und Multiplikativakkumulieren : $mac_m$* umfassen, wobei die Sensorfaktor-Einschluss-Zielfunktion mathematisch als $O_m = g(N_m) * R_m$ dargestellt wird, wobei *$g(N_m)$* ein Ausdruck ist, der umgekehrt proportional zur Anzahl von Sensoren ist, *$g(N_m) = 1/(N_m)^k$* , k auf der Kritikalität der Vorrichtungsleistung basiert und $R_m$ die Belohnungsfunktion ist.

8. Ein oder mehrere nichtflüchtige maschinenlesbare Informationsspeichermedien nach Anspruch 7, wobei das erzeugte winzige DQN-Modell auf der tragbaren Vorrichtung, die von dem Subjekt getragen wird, zur Echtzeit-Schlussfolgerung von einem von i) Abwesenheit oder Vorhandensein der Schmerzen und ii) dem Schmerzschwerewert eingesetzt wird, wobei die Echtzeit-Schlussfolgerung Folgendes umfasst:

Empfangen der physiologischen Signale, die von dem optimalen Satz von Sensoren erfasst werden;
Vorverarbeiten der empfangenen physiologischen Signale:

Erhalten eines Satzes von Zeitdomänen-Schmerzmerkmalen und eines Satzes von spektralen Schmerzmerkmalen aus den vorverarbeiteten physiologischen Signalen; und
Vorhersagen von einem von der Abwesenheit oder dem Vorhandensein der Schmerzen und dem Schmerzschwerewert für das Subjekt.

9. Ein oder mehrere nichtflüchtige maschinenlesbare Informationsspeichermedien nach Anspruch 8, umfassend das Auslösen eines ersten Rückkopplungsmechanismus zum Anpassen einer Schmerzmedikationsdosierung, die an das Subjekt abgegeben wird, gemäß dem Schmerzschwerewert und Erzeugen einer Alarmbenachrichtigung, die an eine Vorrichtung eines klinischen Administrators geteilt wird, wenn der Schmerzschwerewert über einem vordefinierten Schmerzschwellenwert liegt, wobei die Schmerzmedikationsdosierung gemäß der folgenden Gleichung berechnet wird:

*Dosierung von Schmerzmitteln = px_n * [gesamt_Dosierung]*, wobei *px_n* ein normalisierter Schmerzwert ist, der basierend auf dem Schmerzschwerewert, dem maximalen und minimalen Wert, die für den Schmerzschwerewert eingestellt sind, berechnet wird.

**10.** Ein oder mehrere nichtflüchtige maschinenlesbare Informationsspeichermedien nach Anspruch 9, umfassend das Auslösen eines zweiten Rückkopplungsmechanismus zum Auslösen eines Schmerzlinderungsstimulationssignals oder einer verbalen Linderung für das Subjekt, wobei das Schmerzlinderungsstimulationssignal gemäß dem normalisierten Schmerzschwerewert basierend auf der Gleichung

$$\textit{Ausgelöstes Schmerzlinderungsstimulationssignal = px\_n * [Eingabe\_Spannung]}$$

**Revendications**

**1.** Procédé mis en œuvre par processeur (200) pour l'évaluation de la douleur, le procédé comprenant :

la détermination (202), par un ou plusieurs processeurs matériels, pour un dispositif portable porté par un sujet, de contraintes matérielles et d'un ensemble de capteurs sur dispositif parmi une pluralité de capteurs identifiés pour détecter des signaux physiologiques pour l'évaluation de la douleur d'un type de douleur pré-identifié associé au sujet,

la réception (204), par un ou plusieurs processeurs matériels, d'un modèle Deep Q-Network (DQN) construit pour détecter la douleur et un score de gravité de la douleur pour le type de douleur pré-identifié, dans lequel le modèle DQN est entraîné en utilisant un ensemble de données d'entraînement comprenant la fusion de données physiologiques acquises à partir de la pluralité de capteurs attachés à chacun d'une pluralité de sujets ;

la génération (206), par un ou plusieurs processeurs matériels, d'un modèle DQN minuscule pour l'évaluation de la douleur à déployer sur le dispositif portable en optimisant le modèle DQN, dans lequel le modèle DQN minuscule est généré conformément à une fonction objective incluant un facteur de capteur ($O_m$) et un espace d'action de recherche d'architecture de réseau neuronal (NN) spécifie un nombre de capteurs ($N_m$) et une combinaison de capteurs sélectionnés parmi la pluralité de capteurs sur dispositif dans chaque épisode en tant que partie de l'espace d'action de recherche d'architecture NN,

dans lequel la fonction objective incluant un facteur de capteur permet la sélection d'un nombre optimal de capteurs sur dispositif et d'une combinaison de capteurs unique qui est un sous-ensemble de l'ensemble de capteurs sur dispositif pour le type de douleur pré-identifié conjointement avec les contraintes matérielles à partir de l'espace d'action de recherche d'architecture NN, et

dans lequel la fonction objective incluant un facteur de capteur est basée sur le nombre de capteurs ($N_m$) sélectionnés dans chaque épisode, et une fonction de récompense ($R_m$) définie comme une fonction de métriques de performance pondérées comprenant, $P=\{Précision : a_m, Taille\ de\ modèle : s_m, Mémoire\ de\ crête : m_m,\ et\ Multiplieur\text{-}accumulateur : mac_m$, dans lequel la fonction objective incluant un facteur de capteur est mathématiquement représentée comme $O_m = g(N_m) * R_m$ , où $g(N_m)$ est une expression inversement proportionnelle au nombre de capteurs, $g(N_m) = 1/(N_m)^k$ , k est basé sur la criticité de la puissance de dispositif, et $R_m$ est la fonction de récompense.

**2.** Procédé selon la revendication 1, dans lequel le modèle DQN minuscule généré est déployé sur le dispositif portable porté par le sujet pour l'inférence en temps réel de l'un parmi i) l'absence ou la présence de la douleur et ii) le score de gravité de la douleur, l'inférence en temps réel comprenant :

la réception des signaux physiologiques capturés par l'ensemble optimal de capteurs ;
le prétraitement des signaux physiologiques reçus :

l'obtention d'un ensemble de caractéristiques de douleur dans le domaine temporel et d'un ensemble de caractéristiques de douleur spectrales à partir des signaux physiologiques prétraités ; et
la prédiction de l'un parmi l'absence ou la présence de la douleur et le score de gravité de la douleur pour le sujet.

**3.** Système (100) pour l'évaluation de la douleur, le système (100) comprenant :

une mémoire (102) stockant des instructions ;
une ou plusieurs interfaces d'entrée/sortie (E/S) (106) ; et
un ou plusieurs processeurs matériels (104) couplés à la mémoire (102) via les une ou plusieurs interfaces E/S (106), dans lequel les un ou plusieurs processeurs matériels (104) sont configurés par les instructions pour :

déterminer, pour un dispositif portable (112) porté par un sujet, des contraintes matérielles et un ensemble de capteurs sur dispositif parmi une pluralité de capteurs identifiés pour détecter des signaux physiologiques pour l'évaluation de la douleur d'un type de douleur pré-identifié associé au sujet,

recevoir un modèle Deep Q-Network (DQN) construit pour détecter la douleur et un score de gravité de la douleur pour le type de douleur pré-identifié, dans lequel le modèle DQN est entraîné en utilisant un ensemble de données d'entraînement comprenant la fusion de données physiologiques acquises à partir de la pluralité de capteurs attachés à chacun d'une pluralité de sujets ; et

générer un modèle DQN minuscule pour l'évaluation de la douleur à déployer sur le dispositif portable en optimisant le modèle DQN, dans lequel le modèle DQN minuscule est généré conformément à une fonction objective incluant un facteur de capteur (Om) et un espace d'action de recherche d'architecture de réseau neuronal (NN) qui incluent un nombre de capteurs (Nm) et une combinaison de capteurs sélectionnés parmi l'ensemble de capteurs sur dispositif dans chaque épisode en tant que partie de l'espace d'action de recherche d'architecture NN,

dans lequel la fonction objective incluant un facteur de capteur permet la sélection d'un nombre optimal de capteurs sur dispositif et d'une combinaison de capteurs unique qui est un sous-ensemble de l'ensemble de capteurs sur dispositif pour le type de douleur pré-identifié conjointement avec les contraintes matérielles à partir de l'espace d'action de recherche d'architecture NN, et

dans lequel la fonction objective incluant un facteur de capteur est basée sur le nombre de capteurs $(N_m)$ sélectionnés dans chaque épisode, et une fonction de récompense $(R_m)$ définie comme une fonction de métriques de performance pondérées comprenant, P={Précision : $a_m$, Taille de modèle : $s_m$, Mémoire de crête : $m_m$, et Multiplieur-accumulateur : $mac_m$, dans lequel la fonction objective incluant un facteur de capteur est mathématiquement représentée comme $O_m = g(N_m) * R_m$, où $g(N_m)$ est une expression inversement proportionnelle au nombre de capteurs, $g(N_m) = 1/(N_m)^k$, k est basé sur la criticité de la puissance de dispositif, et Rm est la fonction de récompense.

4. Système selon la revendication 3, dans lequel les un ou plusieurs processeurs matériels (104) sont configurés pour déployer le modèle DQN minuscule généré sur le dispositif portable (112) porté par le sujet pour l'inférence en temps réel de l'un parmi i) l'absence ou la présence de la douleur et ii) le score de gravité de la douleur, dans lequel l'inférence en temps réel comprend :

la réception des signaux physiologiques capturés par l'ensemble optimal de capteurs ;
le prétraitement des signaux physiologiques reçus :

l'obtention d'un ensemble de caractéristiques de douleur dans le domaine temporel et d'un ensemble de caractéristiques de douleur spectrales à partir des signaux physiologiques prétraités ; et
la prédiction de l'un parmi l'absence ou la présence de la douleur et le score de gravité de la douleur pour le sujet.

5. Système selon la revendication 4, dans lequel les un ou plusieurs processeurs matériels (104) sont configurés pour déclencher un premier mécanisme de rétroaction pour ajuster un dosage de médicament contre la douleur administré au sujet conformément au score de gravité de la douleur et générer une notification d'alerte partagée à un dispositif d'un administrateur clinique si le score de gravité de la douleur est supérieur à un seuil de douleur prédéfini, dans lequel le dosage de médicament contre la douleur est calculé conformément à l'équation :
dosage de médicament contre la douleur = px_n * [totale_dosage], dans lequel px_n est un score de douleur normalisé calculé sur la base du score de gravité de la douleur, des valeurs maximale et minimale définies pour le score de gravité de la douleur.

6. Système selon la revendication 4, dans lequel les un ou plusieurs processeurs matériels (104) sont configurés pour déclencher un second mécanisme de rétroaction pour actionner un signal de stimulation de soulagement de la douleur ou un soulagement verbal au sujet, dans lequel le signal de stimulation de soulagement de la douleur est conforme au score de gravité de la douleur normalisé sur la base de l'équation
Signal de stimulation de soulagement de la douleur actionné = px_n * [entrée tension].

7. Un ou plusieurs supports de stockage d'informations lisibles par machine non transitoires comprenant une ou plusieurs instructions qui, lorsqu'elles sont exécutées par un ou plusieurs processeurs matériels, amènent :

la détermination, pour un dispositif portable porté par un sujet, de contraintes matérielles et d'un ensemble de capteurs sur dispositif parmi une pluralité de capteurs identifiés pour détecter des signaux physiologiques pour l'évaluation de la douleur d'un type de douleur pré-identifié associé au sujet,

la réception d'un modèle Deep Q-Network (DQN) construit pour détecter la douleur et un score de gravité de la douleur pour le type de douleur pré-identifié, dans lequel le modèle DQN est entraîné en utilisant un ensemble de données d'entraînement comprenant la fusion de données physiologiques acquises à partir de la pluralité de capteurs attachés à chacun d'une pluralité de sujets ;

la génération d'un modèle DQN minuscule pour l'évaluation de la douleur à déployer sur le dispositif portable en optimisant le modèle DQN, dans lequel le modèle DQN minuscule est généré conformément à une fonction objective incluant un facteur de capteur (Om) et un espace d'action de recherche d'architecture de réseau neuronal (NN) spécifie un nombre de capteurs (Nm) et une combinaison de capteurs sélectionnés parmi la pluralité de capteurs sur dispositif dans chaque épisode en tant que partie de l'espace d'action de recherche d'architecture NN,

dans lequel la fonction objective incluant un facteur de capteur permet la sélection d'un nombre optimal de capteurs sur dispositif et d'une combinaison de capteurs unique qui est un sous-ensemble de l'ensemble de capteurs sur dispositif pour le type de douleur pré-identifié conjointement avec les contraintes matérielles à partir de l'espace d'action de recherche d'architecture NN, et

dans lequel la fonction objective incluant un facteur de capteur est basée sur le nombre de capteurs $(N_m)$ sélectionnés dans chaque épisode, et une fonction de récompense $(R_m)$ définie comme une fonction de métriques de performance pondérées comprenant, $P=\{Précision : a_m, Taille$ de $modèle : s_m, Mémoire$ de $crête : m_m, et Multiplieur\text{-}accumulateur : mac_m$, dans lequel la fonction objective incluant un facteur de capteur est mathématiquement représentée comme $O_m = g(N_m) * R_m$, où $g(N_m)$ est une expression inversement proportionnelle au nombre de capteurs, $g(N_m) = 1/(N_m)^k$, k est basé sur la criticité de la puissance de dispositif, et Rm est la fonction de récompense.

8. Un ou plusieurs supports de stockage d'informations lisibles par machine non transitoires selon la revendication 7, dans lequel le modèle DQN minuscule généré est déployé sur le dispositif portable porté par le sujet pour l'inférence en temps réel de l'un parmi i) l'absence ou la présence de la douleur et ii) le score de gravité de la douleur, l'inférence en temps réel comprenant :

la réception des signaux physiologiques capturés par l'ensemble optimal de capteurs ;
le prétraitement des signaux physiologiques reçus :

l'obtention d'un ensemble de caractéristiques de douleur dans le domaine temporel et d'un ensemble de caractéristiques de douleur spectrales à partir des signaux physiologiques prétraités ; et
la prédiction de l'un parmi l'absence ou la présence de la douleur et le score de gravité de la douleur pour le sujet.

9. Un ou plusieurs supports de stockage d'informations lisibles par machine non transitoires selon la revendication 8, comprenant le déclenchement d'un premier mécanisme de rétroaction pour ajuster un dosage de médicament contre la douleur administré au sujet conformément au score de gravité de la douleur et générer une notification d'alerte partagée à un dispositif d'un administrateur clinique si le score de gravité de la douleur est supérieur à un seuil de douleur prédéfini, dans lequel le dosage de médicament contre la douleur est calculé conformément à l'équation : *dosage de médicament contre la douleur = px_n * [totale_dosage]*, dans lequel *px_n* est un score de douleur normalisé calculé sur la base du score de gravité de la douleur, des valeurs maximale et minimale définies pour le score de gravité de la douleur.

10. Un ou plusieurs supports de stockage d'informations lisibles par machine non transitoires selon la revendication 9, comprenant le déclenchement d'un second mécanisme de rétroaction pour actionner un signal de stimulation de soulagement de la douleur ou un soulagement verbal au sujet, dans lequel le signal de stimulation de soulagement de la douleur est conforme au score de gravité de la douleur normalisé sur la base de l'équation

*Signal de stimulation de soulagement de la douleur actionné = px_n * [entrée_tension]*

System
100

Wearable
device
112

Feedback
mechanism
114

Processor(s) 104

I/O Interface(s) 106

Memory 102

Database 108

Modules 110
(Tiny DQN model generator)

FIG. 1A

**FIG. 1B**

200

determining, for a wearable device worn by a subject, hardware constraints and a set of on device sensors from among a plurality of sensors identified for sensing physiological signals for assessment of pain of a preidentified pain type associated with the subject — 202

receiving a Deep Q-Network (DQN) model built for detecting the pain and a pain severity score for the preidentified pain type using a training dataset set comprising fusion of physiological data acquired from the plurality of sensors attached to each of a plurality of subjects — 204

generating a tiny DQN model for pain assessment by optimizing the DQN model to be deployed on the wearable device, wherein the tiny DQN model is generated in accordance with a sensor factor inclusive objective function and a NN architecture search action space that include number of sensors and sensor combination selected from among the set of on device sensors in each episode as a part of NN architecture search action space — 206

**FIG. 2**

| EDA | PPG | IMU | EEG | ECG | EMG |

Cumulative Data from all on device sensors of wearable device

Tiny DQN Model Generation: Neural Architecture Search with automated Sensor Selection for wearable device with specific set of sensors

Explore Sensor Combination

Explore NN Layers

Compute Reward

Train and Exploit DQN

tiny DQN model with optimized sensor inputs to be deployed on the wearable device

**FIG. 3**

## REFERENCES CITED IN THE DESCRIPTION

**Patent documents cited in the description**

- IN 202321080711
- US 2023334330 A1